(19)

Europäisches Patentamt
European Patent Office
Office européen des brevets

(11) **EP 4 390 300 A1**

(12) **EUROPEAN PATENT APPLICATION**

(43) Date of publication:
**26.06.2024  Bulletin 2024/26**

(21) Application number: **22383257.7**

(22) Date of filing: **22.12.2022**

(51) International Patent Classification (IPC):
***G01B 9/02015*** (2022.01)   ***G01B 9/02091*** (2022.01)

(52) Cooperative Patent Classification (CPC):
**G01B 9/02028; G01B 9/02091**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Medlumics S.L.**
**28760 Tres Cantos, Madrid (ES)**

(72) Inventors:
  • **DUPERRON, Matthieu**
    **28760 Madrid (ES)**

  • **SANCHO DURÁ, Juan**
    **28760 Madrid (ES)**
  • **ROIGÉ, Abel**
    **28760 Madrid (ES)**
  • **MAS GÓMEZ, Sara**
    **28760 Madrid (ES)**

(74) Representative: **DTS Patent- und Rechtsanwälte Schnekenbühl und Partner mbB**
**Brienner Straße 1**
**80333 München (DE)**

(54) **LONG COHERENCE RANGE OPTICAL ANALYSIS**

(57)   Described herein are systems and methods for optical coherence tomography with a centimetric range of scan depth and a high tolerance of a precision of lengths among different optical components. A system includes a long coherent light source, an optical interfer- ometer with multiple optical components, an optical de- tector with a wide bandwidth, a data acquisition unit with high sampling rate, and a data processing unit to process information of interest.

EP 4 390 300 A1

## Description

FIELD

Embodiments of the present disclosure relate to methods, devices and systems for optical coherence interferometry (such as optical coherence tomography (OCT) and optical coherent refractometry (OCR)) with large range of scan depth and high manufacturing tolerance for performing optical signal analysis and lesion characteristics for ablation.

BACKGROUND

Ablation is a medical technique for producing tissue necrosis. It is used to help treat different pathologies including cancer, Barret's esophagus, or cardiac arrhythmias, among others. In some cases, various energy sources may be utilized for ablation, including pulsed electric fields, cryogenic cooling for cryoablation, radiofrequency, microwave, laser, ultrasound, and the like. The implementation of ablation requires consistent and accurate detection of the tissue to be treated and the surrounding environment. In some cases, optical coherence interferometry, such as OCT or OCR, can be used for measuring various parameters of the tissue samples, such as the tomography and spectra of absorption, reflection, and birefringence. In some cases, these parameters vary during the process of the treatment and requires real time detection and analysis to provide reliable information and guidance to the treatment.

The results of OCT or OCR depend on complicated factors, such as specifications of the light source, quality of the optical paths, quality of the optical contact, local tissue properties, presence of blood flow close to the tissue surface, and so on, and how these factors change in time. Because of the variability of these parameters, it may be difficult to obtain consistent results. Especially, OCT and OCR require effective interference between an optical signal return from the tissue (a sample arm) and another optical signal from a reference optical path (a reference arm). The results of OCT or OCR critically rely on a coherent condition of the two optical signals in the sample arm and the reference arm. For example, a good quality of interference between the two optical signals in the sample arm and the reference arm requires that a length difference between the two optical paths in the sample arm and the reference arm is within a coherence range of the light produced by the light source.

Additionally, OCT or OCR systems can use multiple sample arms (for example, realized by multiple optical fibers) to probe different regions of a tissue, and the optical path of each of the sample arms needs to be comparable with the optical path of the reference arm within a coherence range of the light. If there is a mismatch of coherence between signals in one or more of the sample arms and in the reference arm, the results may be unreliable or inconsistent. Clarity and focus in a multi-fiber system may be maintained by keeping all optical fibers in a set at a very precise length. Yet cutting multiple optical fibers to a precise length, such that there is minimal variability of length between the fibers, is expensive and difficult.

BRIEF SUMMARY

Accordingly, there may be a need for providing new methods, devices, and systems for manufacturing and applying OCT or OCR systems with high tolerance of the lengths of optical fibers, without sacrificing the accuracy of detection of the condition of the tissue samples.

In the embodiments presented herein, methods and systems are described for performing OCR or OCT.

In an embodiment, an example method of performing OCT or OCR is described. The method includes generating a beam of coherent light having a coherence length more than about 1 cm (single path in air) and a wavelength periodically swept within a wavelength range around a central wavelength at a wavelength sweeping frequency, coupling the beam of coherent light into an optical interferometer comprising a reference arm and a sample arm including an optical multiplexer and a plurality of optical paths with a variation of lengths within a range less than the coherence length, selecting an optical path of the plurality of optical paths using the optical multiplexer, processing a plurality of output signals of the optical interferometer, each of the plurality of output signals corresponds to one of the plurality of optical paths. The method further includes acquiring data from the plurality of output signals at a data sampling rate and processing the data and generating a plurality of signals from the data. The plurality of signals have depth ranges of at least 1 cm, and each of the plurality of signals corresponds to one of the plurality of optical paths.

In another embodiment, a system of OCT or OCR is described. The system includes a light source, an optical interferometer, an optical multiplexer, an optical detector, a data acquisition unit, and a data processing unit. The light source is configured to output a beam of coherent light having a coherence length more than about 1 cm (single path in air) and a wavelength periodically swept within a wavelength range around a central wavelength at a wavelength sweeping frequency. The optical interferometer includes an input coupled to the beam of coherent light, an output, a reference arm, and a sample arm including a plurality of optical paths with a variation of lengths within a range less than the coherence length. The optical multiplexer is configured to select one of the plurality of optical paths. The optical detector is coupled to the output of the optical interferometer. The data acquisition unit is configured to acquire data from the optical detector at a data sampling rate. The data processing unit is configured to generate a plurality of signals from the data. The plurality of signals have depth ranges of at least 1 cm, each corresponds to one of the plurality of optical paths.

Further features and advantages, as well as the structure and operation of various embodiments, are described in detail below with reference to the accompanying drawings. It is noted that the specific embodiments described herein are not intended to be limiting. Such embodiments are presented herein for illustrative purposes only. Additional embodiments will be apparent to persons skilled in the relevant art(s) based on the teachings contained herein.

BRIEF DESCRIPTION OF THE DRAWINGS/FIGURES

The accompanying drawings, which are incorporated herein and form a part of the specification, illustrate embodiments of the present disclosure and, together with the description, further serve to explain the principles of the disclosure and to enable a person skilled in the pertinent art to make and use the disclosure.

    FIG. 1 illustrates a diagram of an example system for ablation, according to embodiments of the present disclosure.
    FIG. 2 illustrates an example diagram of a catheter, according to embodiments of the present disclosure.
    FIGs. 3A and 3B illustrate cross sections of a catheter, according to embodiments of the present disclosure.
    FIG. 4 illustrates a diagram of an example distal section of a catheter, according to embodiments of the present disclosure.
    FIG. 5 illustrates a diagram of an example arrangement of optical fibers in which light beams are directed to different directions at end terminals of optical components of a catheter, according to embodiments of the present disclosure.
    FIG. 6 illustrates a diagram of an example OCT and/or OCR system with multiple optical components, according to embodiments of the present disclosure.
    FIG. 7A and 7B illustrates exemplary diagrams of OCR scans by two different optical components, according to embodiments of the present disclosure.
    FIG. 8A illustrates a diagram of an example of an optical component of an OCT system with a short range of scan depth, according to embodiments of the present disclosure.
    FIG. 8B illustrates a diagram of an example of an optical component of an OCT system with a long range of scan depth, according to embodiments of the present disclosure.
    FIG. 9 illustrates an example graphical user interface (GUI) showing predicted lesion depths probed by different optical components, according to embodiments of the present disclosure.
    FIG. 10 illustrates an example method of conducting OCR with multiple optical components, according to embodiments of the present disclosure.

Embodiments of the present disclosure will be described with reference to the accompanying drawings.

DETAILED DESCRIPTION

Although specific configurations and arrangements are discussed, it should be understood that this is done for illustrative purposes only. A person skilled in the pertinent art will recognize that other configurations and arrangements can be used without departing from the spirit and scope of the present disclosure. It will be apparent to a person skilled in the pertinent art that this disclosure can also be employed in a variety of other applications.

It is noted that references in the specification to "one embodiment," "an embodiment," "an example embodiment," etc., indicate that the embodiment described may include a particular feature, structure, or characteristic, but every embodiment may not necessarily include the particular feature, structure, or characteristic. Moreover, such phrases do not necessarily refer to the same embodiment. Further, when a particular feature, structure or characteristic is described in connection with an embodiment, it would be within the knowledge of one skilled in the art to effect such feature, structure or characteristic in connection with other embodiments whether or not explicitly described.

It should be noted that although this application may refer specifically to cardiac ablation, the embodiments described herein may target other pathologies as well, along with additional energy sources for ablation, including but not limited to cryogenic, radiofrequency (RF), microwave, laser, ultrasound, and pulsed electric fields. The principles of using laser energy to treat other pathologies are similar, and therefore the techniques used to apply the laser energy are similar.

Disclosed herein are embodiments of OCT/OCR systems with an ablation catheter for merged optical tissue evaluation and laser ablation in which the ablation catheter includes a plurality of optical ports for both evaluating and ablating target tissue. In some embodiments, the plurality of optical ports of the catheter may be configured to transmit beams of exposure radiation to a sample, receive one or more beams of scattered radiation that have been reflected or scattered from the sample, and transmit laser energy such that at least a portion of the sample is ablated. By utilizing the same optical ports for transmission of the optical evaluation signals and the laser ablation signals, the ablation catheter may provide focused evaluation of the same target tissue that is being ablated in a single substrate that allows for both modalities.

Herein, the terms "electromagnetic radiation," "light," and "beam of radiation" are all used to describe the same electromagnetic signals propagating through the various described elements and systems.

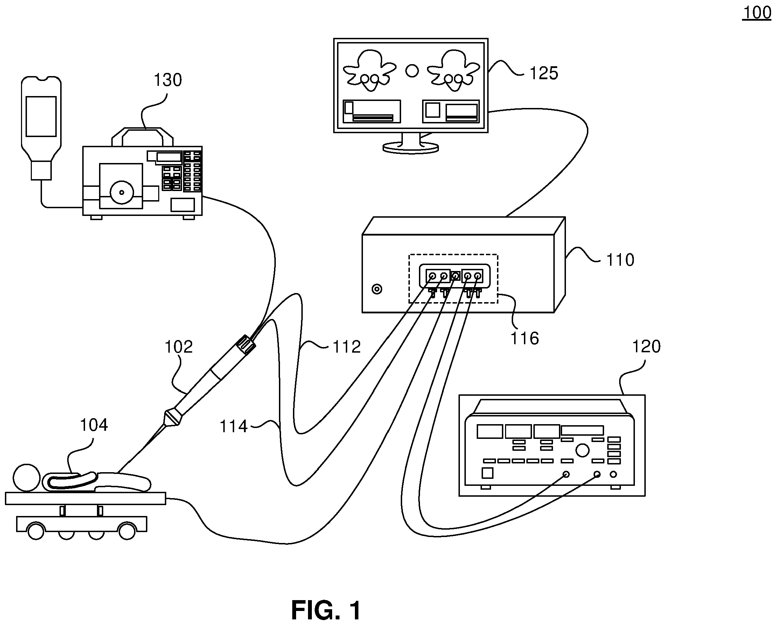

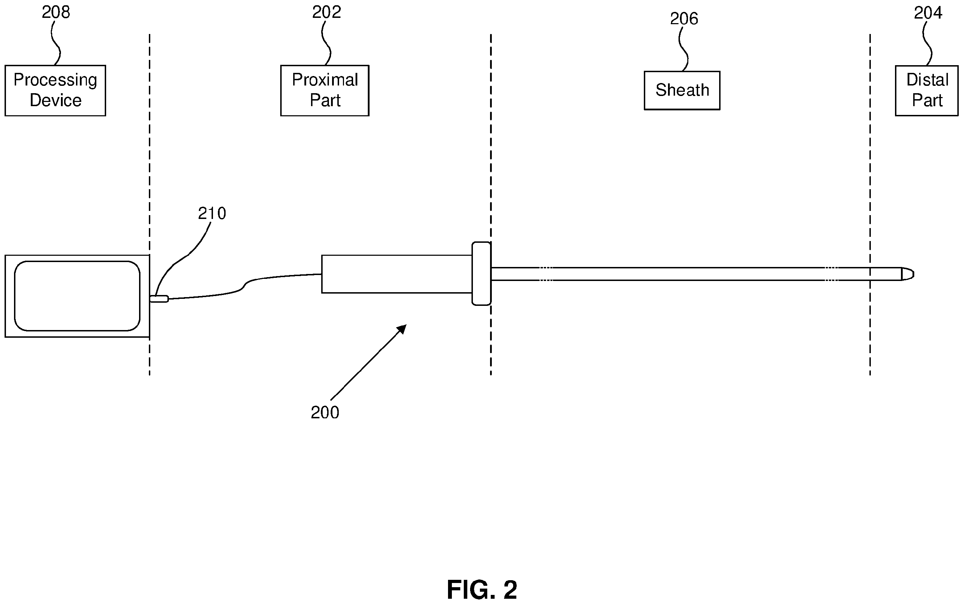

In some embodiments, an ablation catheter and console system described herein uses OCT and/or OCR, or other methods to perform tissue ablations, track formation of scar in real-time, and monitor/verify lesion geometries and isolation by directly observing the scar pattern in tissue. FIG. 1 illustrates a diagram of an example system 100 for performing ablation according to embodiments of the present disclosure. The system 100 includes catheter 102, console 110, signal generator 120, display 125, and irrigation pump 130. The catheter 102, console 110, signal generator 120, display 125, and irrigation pump 130 can be communicatively coupled together via wired and/or wireless connections. In some embodiments, catheter 102 can represent an exemplary embodiment of catheter 200 shown in FIG. 2. In some embodiments, patient 104 is shown in FIG. 1 for illustrative purposes. It is understood that the embodiments described herein can be used in vivo and/or in vitro.

In some embodiments, catheter 102 can be positioned at a portion of tissue subject to ablation using energy generated by signal generator 120. In some embodiments, signal generator 120 can be an electronic device configured to generate radiofrequency (RF), cryogenic, or electroporation (e.g., pulsed electric field) signals for ablation. Signal generator 120 can be coupled to catheter 102 directly or via the console 110, and can send energy to catheter 102 to ablate the portion of tissue at a selected tissue site. In some embodiments, the portion of tissue can be myocardial tissue, cardiac muscle tissue, skeletal tissue, or the like. Energy can be applied to the portion of tissue through optical view ports in the distal section of catheter 102. After applying the energy, structural changes in the tissue can be observed by acquiring optical signals via one or more optical view ports of catheter 102.

Console 110 can comprise a computing device configured to acquire the optical signals from catheter 102 and analyze the optical signals to detect changes in optical properties of the tissue. In some embodiments, console 110 can include hardware (e.g., circuits), firmware, software, or any combination thereof to process the optical signals and perform further analysis. In some embodiments, console 110 can include an internal light source that provides a beam of coherent light. In some embodiments, console 110 can be coupled to an external light source that provides the beam of coherent light. In some embodiments, console 110 can send the beam of coherent light through an optical circuit within itself and the catheter 102 and into the tissue to monitor scar progression, contact condition between the tissue and catheter 102, and other characteristics of the tissue.

In some embodiments, console 110 can be referred to herein as a control console, a processing device, and/or controller. Console 110 may be coupled to display 125, which can present results from the optical signal analysis and allow a user to select/view, modify, and/or control parameters related to operation of catheter 102, console 110, signal generator 320, and/or irrigation pump 330.

In some embodiments, irrigation pump 130 may be coupled to catheter 102 via a tubing. In some embodiments, irrigation pump 130 may allow for fluid to be pumped through the tubing and released at the tissue site through catheter 102 (e.g., through optical view ports or through separate irrigation slits at the distal section of catheter 102). Fluid from the irrigation pump 130 may cool the distal section of catheter 102 and the surrounding tissue during ablation, and also flush away any debris during and/or after ablation.

In some embodiments, catheter 102 may be coupled to console 110 via one or more optical connections 112 and one or more electrical connections 114. Optical connections 112 may include single mode optical fibers and/or multimode optical fibers that allow acquisition and/or transmission of optical signals to and from catheter 102 and console 110 for further analysis. Electrical connections 114 may include wiring, pins, and/or components used for supplying power and energy from signal generator 120 to catheter 102 for ablation.

In some embodiments, the optical and electrical connections 112, 114 may be connected to console 110 via a communication interface 116. Communication interface 116 may allow for transmission of various signals (e.g., optical and electrical signals) between catheter 102 and console 110. In some embodiments, the communication interface 116 may include a connector that facilitates proper alignment of optical fibers between the catheter 102 and console 110.

FIG. 2 illustrates a catheter 200 according to embodiments of the present disclosure. Catheter 200 includes a proximal section 202, a distal section 204, and a shaft 206 coupled between proximal section 202 and distal section 204. In an embodiment, shaft 206 includes one or more radiopaque markers for navigation purposes. In one embodiment, catheter 200 includes a communication interface 210 between catheter 200 and a processing device 208. Communication interface 210 may include one or more one or more optical fibers and connectors between processing device 208 and catheter 200, as described herein. In other examples, communication interface 210 may include an interface component that allows wireless communication, such as Bluetooth, WiFi, cellular, and the like, to communicate with the catheter 200 or other processing components in a catheter system

In an embodiment, shaft 206 and distal section 204 are disposable. As such, proximal section 202 may be reused by attaching a new shaft 206 and proximal section 204 each time a new procedure is to be performed. In another embodiment, proximal section 202 is also disposable.

Proximal section 202 may house various electrical and optical components used in the operation of catheter 200. In some embodiments, an optical source may be included within proximal section 202 to generate a source beam of radiation for optical evaluation. In some embodiment, the optical source can be an external optical source coupled to the proximal section via optical connections 112 in FIG. 1. The optical source may include one or more lasers, laser diodes, light emitting diodes (LEDs), or other kinds of optical coherent source. The beam of radiation generated by the optical source may have a wavelength within the infrared range. In one example, the beam of radiation has a central wavelength between about 1000 nm and 1600 nm. For example, the beam of radiation may have a central wavelength of 1050 nm, 1310 nm, or 1550 nm. The optical source may be designed to output a beam of radiation at only a single wavelength, or it may be a swept source and be designed to output a range of different wavelengths. The generated beam of radiation may be guided towards distal section 204 via the optical transmission medium connected between proximal section 202 and distal section 204 within shaft 206. Some examples of optical transmission media include single mode optical fibers and/or multimode optical fibers. In one embodiment, the electrical transmission medium and the optical transmission medium are provided by the same hybrid medium allowing for both electrical and optical signal propagation.

Furthermore, proximal section 202 may include another optical source, such as a laser energy source, to generate laser energy that is applied at distal section 204 for tissue ablation. In some embodiments, the laser energy source may emit an ablation beam of laser energy at a wavelength of 980 nm or a wavelength of 1060 nm. The laser energy from the source in the proximal section 202 may propagate down the catheter 200 via an optical transmission medium connected between proximal section 202 and distal section 204 within shaft 206, and the laser energy may be output from the distal section 204 of catheter 200 to target tissue. For example, the laser energy from the source may produce an optical power of 5W to 12W that is applied to target tissue for 20-30 seconds to produce transmural lesions in heart tissue. In another example, the laser energy from the source may produce an optical power of 30W to 50W that is applied to target tissue for 60-90 seconds.

In an embodiment, proximal section 202 includes one or more components of an interferometer in order to perform coherence interferometry using the light generated from the optical sources. Due to the nature of interferometric data analysis, in an embodiment, the optical transmission medium used for guiding the light to and from distal section 204 does not affect the state and degree of light polarization. In another embodiment, the optical transmission medium can affect the polarization in a constant and reversible way. In some embodiments, catheter 200 may include an optical circuit with one or more elements configured to conduct optical spectroscopy. In such embodiments, at least part of the optical path may be made up of multi-mode optical transmission media (e.g. multi-mode optical fiber).

Proximal section 202 may include further interface elements with which a user of catheter 200 can control the operation of catheter 200. For example, proximal section 202 may include a deflection control mechanism that controls a deflection angle of distal section 204. The deflection control mechanism may include a mechanical movement of an element on proximal section 202, or the deflection control mechanism may use electrical connections to control the movement of distal section 204. Proximal section 202 may include various buttons or switches that allow a user to control when laser energy is applied at distal section 204, or when the beams of radiation are transmitted from distal section 204, allowing for the acquisition of optical data. In some embodiments, proximal section 202 may include a deflection control mechanism for controlling one or more pull wires that are coupled to the distal section 204. In some embodiments, deflection control mechanism and the one or more pull wires allow for steering of the distal section of catheter 200 in order to maneuver within and target specific tissue regions for ablation.

Distal section 204 includes a plurality of optical view ports. In some embodiments, the plurality of optical view ports may be referred to herein as orifices in the catheter tip. In an embodiment, one or more of the optical view ports are machined into the outer body of distal section 204. The optical view ports are distributed over the outside of distal section 104, resulting in a plurality of distinct viewing directions. In some embodiments, the optical view ports may transmit and collect light (e.g., optical signals) at various angles from the distal section 204. The optical view ports also allow for a plurality of directions (e.g., beam directions) in which laser energy may be directed for tissue ablation through one or more of the optical view ports. In an embodiment, each of the plurality of viewing directions are substantially non-coplanar. The optical view ports may also be designed with irrigation functionality to cool distal section 204 and surrounding tissue during ablation.

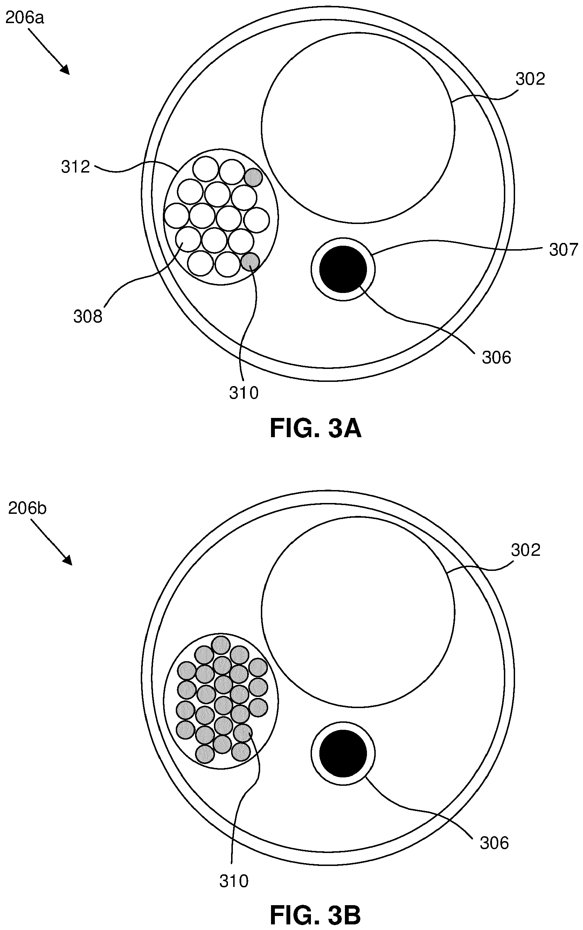

FIGs. 3A and 3B illustrate cross-section views of shaft 206, according to embodiments of the present disclosure. Shaft 206 may include all of the elements interconnecting proximal section 202 with distal section 204. Shaft 206a illustrates an embodiment that houses multiple channels/lumens, including an irrigation channel 302, a cabling channel 312, and a channel for deflection mechanisms 307. Through these channels 307, 312, 302, deflection mechanism 306, electrical connections 308, and optical transmission medium 310, and cooling fluid may be at least partially housed or transported. In some configurations, a protective cover wrapped around both electrical connections 308 and optical transmission media 310 may be used. In other embodiments, optical transmission media 310 and components may be located within a protective cover that is separate from the protective cover in which the electrical connections 308 is housed. Electrical connections 308 may be used to provide signals to optical modulating components located in distal section 204. One or more optical transmission media 310 guide light generated from the optical source (exposure light) towards distal section 204, while another subset of optical transmission media 310 guides light returning from distal section 204 (scattered or reflected light) back to proximal section 202. In another example, the same one or more optical transmission media 310 guides light in both directions. In some embodiments, the optical transmission medium 310 comprises one or more single mode optical fibers and/or multimode optical fibers.

Irrigation channel 302 may be a hollow tube used to guide cooling fluid towards distal section 204. Irrigation channel 302 may include heating and/or cooling elements disposed along the channel to affect the temperature of the fluid. In another embodiment, irrigation channel 302 may also be used as an avenue for drawing fluid surrounding distal section 204 back towards proximal section 302.

Deflection mechanism 306 may include electrical or mechanical elements designed to provide a signal to distal section 204 in order to change a deflection angle of distal section 204. The deflection system enables guidance of distal section 204 by actuating a mechanical control placed in proximal section 202, according to an embodiment. This system may be based on a series of aligned and uniformly spaced cutouts in shaft 206 aimed at providing unidirectional deflection of distal section 204, in combination with a wire which connects the deflection mechanism control in proximal section 202 with the catheter tip at distal section 204. In this way, a certain movement of the proximal section may be projected to the distal section. Other embodiments involving the combination of several control wires attached to the catheter tip may enable the deflection of the catheter tip along different directions.

FIG. 3B illustrates a cross-section of shaft 206b. Shaft 206b depicts an embodiment having most of the same elements as shaft 206a from FIG. 3A, except that there are no electrical connections 308. Shaft 206b may be used in situations where modulation (e.g., multiplexing) of the generated beam of radiation is performed in proximal section 202.

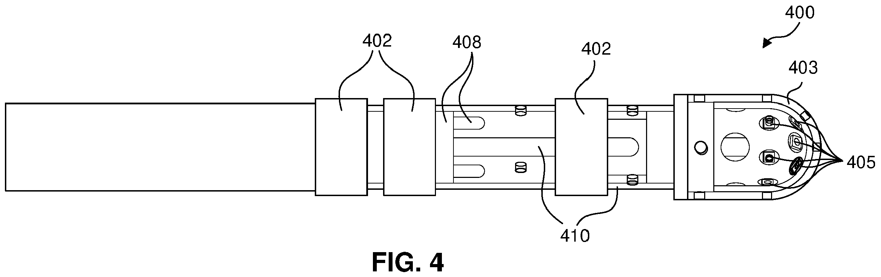

FIG. 4 illustrates a diagram of an example distal section of catheter 400, according to embodiments of the present disclosure. In some embodiments, the distal section of catheter 400 in FIG. 4A may represent an exemplary embodiment of distal section 204 of catheter 200 shown in FIG. 2. The distal section of catheter 400 includes a plurality of electrodes 402, ablation cap 403, a plurality of optical ports 405, one or more pull wire components 408, and irrigation tubing 410. In some embodiments, ablation cap 403 may also be an electrode and may be metallic. In some embodiments, ablation cap 403 may be referred to as a distal cap. In some embodiments, the plurality of optical ports 405 may be referred to herein as a plurality of optical view ports. In some embodiments, the pull wire components 408 may include an anchor and/or other components for allowing steering of the distal section of catheter 400 in order to maneuver within and target specific tissue regions for ablation. In some embodiments, irrigation tubing 410 may allow fluid to be guided along the catheter tip to cool tissue.

In some embodiments, the ablation cap 403 may include multiple optical ports 405, which may serve as orifices for optical ablation and also as optical windows or view ports for light beams from a plurality of optical fibers in the catheter. In some embodiments, optical ports 405 can be distributed in a non-planar manner on a dome-shaped surface of ablation cap 403. In some embodiments, optical fibers (for example, optical transmission media 310 in FIGs. 3A and 3B) may be directed through the catheter shaft to lenses on the distal section of the catheter. In some embodiments, the optical fibers may be connected to the lenses by wafer-based wave-guide circuits that define the optical components at the catheter tip. In other embodiments, the optical fibers in the catheter tip may connect directly to the lenses, which focus the light into the tissue through the plurality of optical ports 405. In some embodiments, the lenses may be silicon or formed from another optically transparent material. In some embodiments, the lenses may also be coated to reduce reflections at interfaces or to allow optical index differences with surrounding tissue, blood, or fluid media.

In some embodiments, the catheter tip may include passive and fixed number of optics components (e.g., 15 fibers with 15 lenses attached), without any mechanical switching or scanning devices in the catheter itself. In some embodiments, movement or rotation of optical elements may allow for scanning in different directions in the tissue. In some embodiments, the plurality of optical ports or view ports in the catheter may have various orientations in the catheter tip, in which each output beam directed from each view port in the catheter may face a different direction. For example, one output beam may be directed forward, seven output beams may be directed at 45° with respect to tissue, and seven output beams may be directed at 90° with respect to tissue. In some embodiments, there may be any number of beams, view ports, orientations of the view ports in the catheter tip.

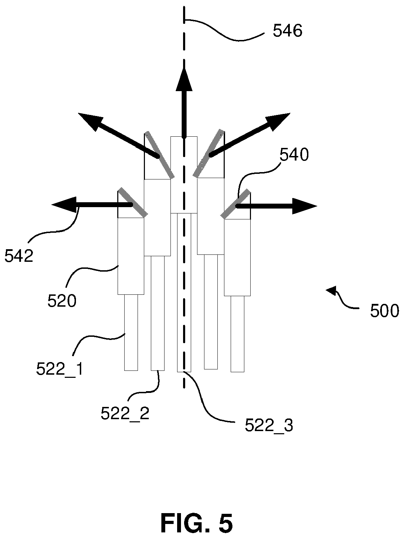

FIG. 5 illustrates a diagram of an example arrangement 500 of optical fibers in which light beams are directed to different directions at end terminals of optical components of a catheter, according to embodiments of the present disclosure. In some embodiments, arrangement 500 can be located in a distal section of the catheter. FIG. 5 includes the arrangement of five optical fibers 522, each connected to an optical head at an end terminal of the optical fiber. For example, optical head 520 is connected at an end terminal of optical fibers $522_1$. Each of the optical heads corresponds to one of optical ports 405 in FIG. 4. Optical head 520 can include optical elements to couple optical signals in and out of optical fibers $522_1$. For example, optical head 520 can include one or more optical lenses to focus an output beam of optical fibers $522_1$. In some embodiments, mirrors can be adapted to each of the optical heads to direct the output beams to a certain orientation. For example, mirror 524 adapted to optical head 520 can direct the output beam 542 at 90° with respect to the optical head. In some embodiments, mirrors adapted to optical heads can direct output beams to other directions, for example, 30°, 45°, or 60° with respect to the optical heads. In some embodiment, the orientation of mirror 524 can be mechanically controlled, such that the output beam 542 can be directed to an arbitrary angle. For example, mirror 542 can be controlled by deflection mechanism 306 in FIG. 3. In some embodiments, optical head 520 may not be equipped with mirror 524. For example, in FIG. 5, the optical head along a cylindrical axis of the catheter (axis 546) is not equipped with mirror 524, such that the output beam of this specific optical head is transmitted along axis 546. Although FIG. 5 includes the arrangement of five optical fibers, it should be understood that this is for illustrative purpose only. A person skill in the art will recognize that other quantity of optical fibers and their arrangements can be used without departing from the spirit and scope of the present disclosure.

Since optical ports 405 are distributed at different locations at the end of the distal section of catheter 400, adapting optical heads 520 to optical ports 405 may require that end terminals of optical fibers 522 are not aligned, resulting in different lengths for different optical fibers 522. This can cause variation of lengths of optical paths in different optical components, and variation of the quality of interference between each of the optical components and a reference arm. If such variation is comparable or greater than a coherence length of the light, the reliability and consistency of optical signals collected by the optical components are affected. For example, in some embodiments, the difference of length between an optical component (sample arm) and the reference arm must be adjusted with a sub-millimetric precision to ensure a scan depth range of a few millimeters in the sample. Each fiber is typically several meters long, yet typically needs its length to be controlled with a sub-millimetric precision. Such a high demanding precision of fiber lengths imposes extra difficulties in manufactoring and increases the complexity and time of assembly, and the cost of the products, especially when one or more fibers of a catheter need to be replaced.

In embodiments described herein, the tolerance of the precision of fiber lengths can be relaxed by using an optical coherent source having a long coherence length in conjunction with a high sampling frequency such that a sufficient number of samples are taken across a depth range. The regions of interest are then identified and cropped in the returned signal for processing and analysis. For example, a swept source with a tunable wavelength (for example, via a MEMS tunable filter), an akinetic swept source, or a vertical-cavity surface-emitting laser (VCSEL) with a tunable wavelength (for example, via a MEMS tunable filter), can provide a centimetric range of coherence length. A longer coherence length of the optical source can provide the opportunity for larger scan depth range, but also requires adequate design of acquisition system for detecting, sampling, and processing the optical signals. For example, in one embodiment, for a swept-source OCT system, obtaining a 1 cm imaging range out of a 100 nm wavelength sweeping range laser may use an acquisition sampling rate at least 2000 times faster than the swept source frequency (e.g. if the swept-source frequency is 50 kHz, the detector bandwidth would be higher than 100 MHz). Therefore, the acquisition system is optimized for a sampling frequency high enough to allow centimetric depth measurement. To support the larger scan depth range, a data processing unit may compute the A-scan of the full centimetric depth range and individually select the region of interest in the optical signal for each optical component. In some embodiments, for example, detecting and cropping the region of interest can be done by peak-detection or pre-calibration.

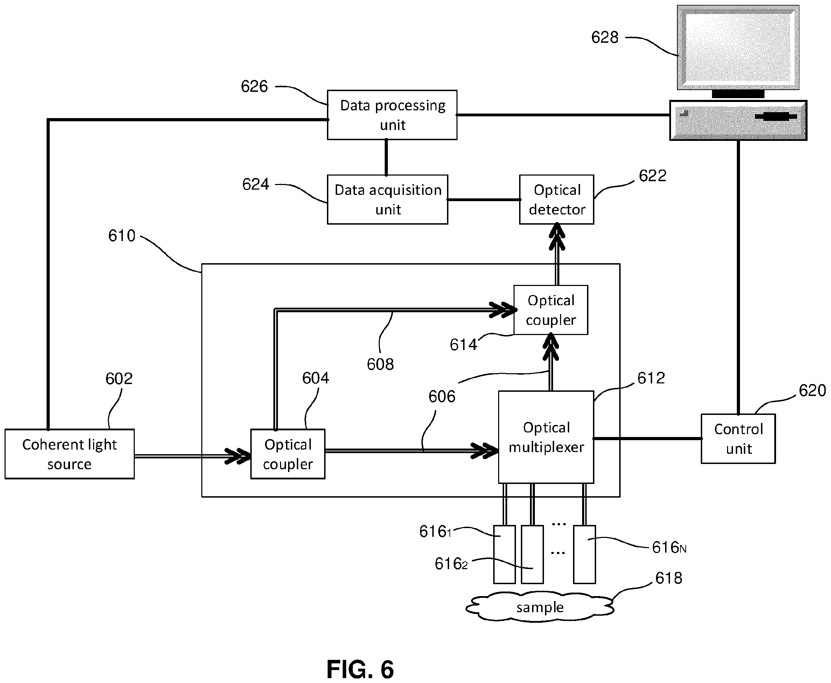

FIG. 6 illustrates a diagram of an example OCT and/or OCR system with multiple optical components, according to embodiments of the present disclosure. A coherent light source 602 provides a beam of coherent light with a centimetric range of coherence length. In some embodiments, coherent light source 602 can be a swept source with tunable wavelength (for example, via a MEMS tunable filter), an akinetic swept source, or a VCSEL with tunable wavelength (for example, via a MEMS tunable filter). In some embodiments, coherent light source 602 can provide a beam of coherent light with a coherence length of several centimeters (single path in air). For example, the beam of coherent light provided by coherent light source 602 can have a coherence length of 1.0 cm, 2.4 cm, 5 cm, 8 cm, or 10 cm. In some embodiments, coherent light source 602 can provide a beam of coherent light with a coherence length more than about 1 cm. For example, the beam of coherent light provided by coherent light source 602 can have a coherence length of 10 cm, 15 cm, 20 cm, and 30 cm. In some embodiments, coherent light source 602 can be a sweep source that generates a beam of coherent light having a wavelength periodically swept within a wavelength range around a central wavelength at a wavelength sweeping frequency. In some embodiments, the central wavelength can be infrared. In some embodiments, the central wavelength can be between 1000 nm and 1600 nm. For example, the central wavelength of the beam of coherent light can be about 1310 nm. In some embodiments, the wavelength range swept around the central wavelength can be between 30 nm and 70 nm. For example, the wavelength range swept around the central wavelength can be about 55 nm. In some embodiments, the wavelength sweeping frequency can be between 20 kHz and 100 kHz. For example, the wavelength sweeping frequency can be about 50 kHz. In another example, the wavelength sweeping frequency can be about 30 kHz. In some embodiments, coherent light source 602 can be an internal light source included in console 110 (as shown in FIG. 1) and coupled to optical transmission medium 310 (as shown in FIGs. 3A and 3B) of the catheter. In some embodiments, coherent light source 602 can be an external light source coupled to the catheter via console 110.

The beam of coherent light generated by coherent light source 602 is coupled to an interferometer 610. Interferometer 610 includes a first optical coupler 604 that couples a first portion of the beam of coherent light to a reference arm 608 and a second portion of the beam of coherent light to a sample arm 606. Sample arm 606 is coupled to an optical multiplexer 612, which guides the second portion of the beam of coherent light into one or more optical components $616_1$ - $616_N$. Each of optical components $616_1$ - $616_N$ includes an optical path that guides the second portion of the beam of coherent light to a location on a sample 618 to be inspected. Sample 618 can be a lesion, a tissue under an ablation process, or a test sample. Each of optical components $616_1$ - $616_N$ can deliver the second portion of the beam of coherent light through one of the optical ports at the distal section of the catheter (for example, optical ports 405 in FIG. 4). After illumination by the coherent light, a plurality of optical signals can return from sample 618, each corresponding to optical components $616_1$ - $616_N$. The plurality of optical signals depends on the condition of sample 618 and are correlated to the beam of coherent light. The plurality of optical signals are collected back by optical components $616_1$ - $616_N$ and returned to sample arm 606 via optical multiplexer 612, and then combined with the first portion of the beam of coherent light in reference arm 608 by a second optical coupler 614. In some embodiments, reference arm 608 can include an optical fiber with an optical path of a certain length. In some embodiments, a control unit 620 can be used to control optical multiplexer 612 to select one or more optical components $616_1$ - $616_N$, to which the second portion of the beam of coherent light is coupled. Control unit 620 can be connected to a computer 628 to receive commands from and transmit data to computer 628.

In some embodiments, each of optical components $616_1$ - $616_N$ can include an optical fiber adapted to an optical head (for example, optical fiber 522 and optical head 520 as shown in FIG. 5). In some embodiments, the lengths of optical paths of optical components $616_1$ - $616_N$ can be different. In order to provide effective interference between reference arm 608 and sample arm 606, the lengths of optical paths of optical components $616_1$ - $616_N$ need to be comparable with the length of the optical path of reference arm 608 within the coherence length of the beam of coherent light, and a variation of lengths of optical paths among optical components $616_1$ - $616_N$ need to be shorter than the coherence length of the beam of coherent light. For example, if the beam of coherent light generated by coherent light source 602 has a coherence length of about 2.4 cm (single path in air), the variation of lengths of optical paths between optical components $616_1$ - $616_N$ can be within a range less than about 2.4 cm. For example, optical components $616_1$ - $616_N$ can have a maximum length difference of about 6mm (double path in fiber), corresponding to about 17 mm in air (single path).

In some embodiments, optical multiplexer 612 and optical components $616_1$ - $616_N$ can be included inside catheter 102 of FIG. 1. In some embodiments, optical multiplexer 612 can be located inside console 110 of FIG. 1, and coupled to optical components $616_1$ - $616_N$ in catheter 102 via optical connections 112. In some embodiments, the first optical coupler 604, the second optical coupler 614, and the reference arm 608 can be included in console 110.

Interferometer 610 provides a plurality of optical output signals by second optical coupler 614, each corresponding to one of optical components $616_1$ - $616_N$. Each of the optical output signals includes information about the interference between the reference arm and the sample arm coupled to one of optical components $616_1$ - $616_N$. The optical output signals are detected by an optical detector 622. Optical detector 622 can convert the optical output signals into electrical signals containing information about the interference between the reference arm and the sample arm coupled to one of optical component $616_1$ - $616_N$, as functions of time. Since the wavelength of the beam of coherent light is being swept within a wavelength range under a sweeping frequency, the wavelength is also a function of time. Therefore, within each cycle of the wavelength sweeping, each of the electrical signals provided by optical detector 622 is also a function of the varying wavelength. A Fourier transform of the electrical signal from a wavelength domain (or equivalently, a wavenumber domain) into a real space domain (or equivalently, a depth domain) can provide signals related to optical properties of a location of sample 618 probed by an optical component, as functions of scan depth.

In some embodiments, optical detector 622 can process the optical output signals provided by interferometer 610 for effective measurement. For example, optical detector 622 can amplify the optical output signals, or filter the optical output signals to remove noise. In order to avoid signal distortion and provide high quality electrical signals containing information about sample 618, optical detector 622 should have a sufficient bandwidth to process and detect the optical output signals. In some embodiments, optical detector 622 can have a bandwidth of about 100 MHz.

In some embodiments, optical detector 622 can be located inside console 110 in FIG. 1, and the optical output signals can be transmitted to optical detector 622 via optical connections 112 and interface 116. In some embodiments, optical detector 622 can be located inside catheter 102 in FIG. 1, and the electrical signals provided by optical detector 622 can be transmitted to console 110 via electrical connections 114 and interface 116.

Electrical signals provided by optical detector 622 can further be sampled into digital data by a data acquisition unit 624, at a data sampling rate. In some embodiments, data acquisition unit 624 can be located inside console 110 in FIG. 1. In order to avoid signal distortion or loss of information in the signal, the data sampling rate of data acquisition unit 624 can be sufficiently higher than the bandwidth of the electrical signals provided by optical detector 622. In some embodiments, the data sampling rate can be greater than the bandwidth of optical detector. For example, the data sampling rate can greater than 100 MHz. In some embodiments, the data sampling rate can be between 0 and 175 MHz. In some embodiments, the data sampling rate can be between 0 and 400 MHz. In some embodiments, the data sampling rate of data acquisition unit 624 can be set by the k-clock of coherent light source 602 (for example, the k-clock of a VCSEL swept laser), such that the overall image range (for example, 12 mm double path in air) can automatically be ensured, and any sweeping nonlinearity can be compensated.

The data acquired by data acquisition unit 624 is further transmitted to data processing unit 626. In some embodiments, data processing unit 626 can be located in console 110 in FIG. 1. In some embodiments, data processing unit 626 can be a processing unit of computer 628. Data processing unit 626 can process the data and extract useful information from the data that can help assisting the analysis of the condition of sample 618. For example, data processing unit 626 can use fast Fourier transform to convert the data as a function of wavelength into a plurality of signals as functions of scan depth from the surface of sample 618, with each of the plurality of signals corresponding to the condition of a location of sample 618 measured by one of the optical components $616_1$ - $616_N$. In some embodiments, data processing unit 626 can also be connected to coherent light source 602 to acquire the phase of the sweeping wavelength, so that the information of the wavelength is collected by data processing unit 626 for applying fast Fourier transform. In some embodiments, the plurality of signals can include information about refraction, birefringent, polarization, and/or other optical properties of sample 618. The plurality of signals as functions of scan depth can further be transmitted to display on a screen of computer 628.

In some embodiments, data processing unit 626 can also process the plurality of signals to extract information of interest in them. For example, data processing unit 626 can detect one or more reference features in the plurality of signals, and crop regions of interest in the plurality of signals, based on the location of one or more reference features.

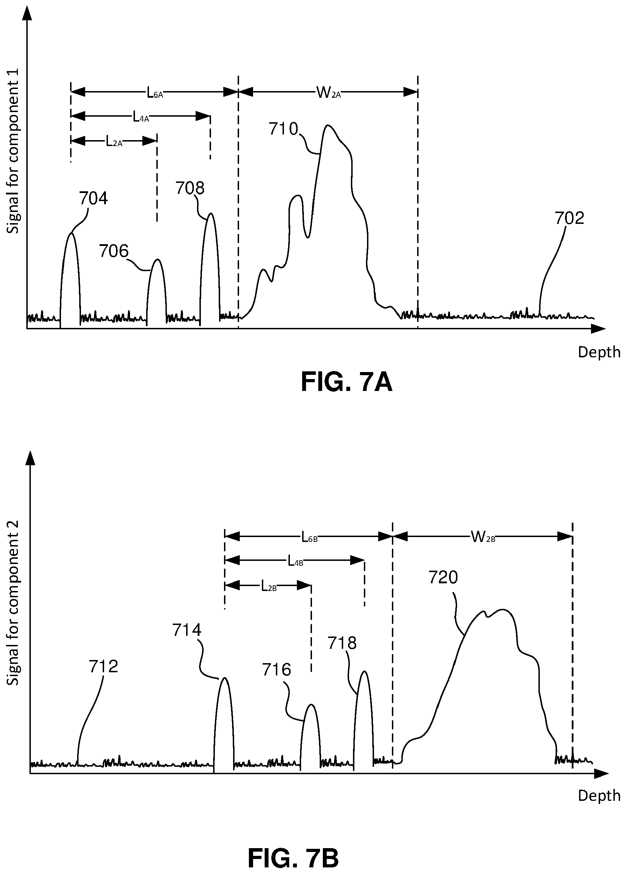

FIGs. 7A and 7B illustrate exemplary diagrams of OCR scans by two different optical components, according to embodiments of the present disclosure. In some embodiments, the range of scan depth of OCR scans can be at least 1 cm. For example, the ranges of scan depth, or the total spans of depth in FIGs. 7A and 7B, can be 1 cm, 2 cm, 5 cm, 8 cm, or 10 cm. In some embodiments, the range of scan depth of OCR scans can be greater than 10 cm.

FIG. 7A illustrates first signals provided by data processing unit 626 after applying fast Fourier transform on data sampled from an electrical signal provided by optical detector 622 in FIG. 6, in which the electrical signal corresponds to an optical signal returned from a first optical component $616_1$ probing a first location of sample 618. A background noise signal 702 in FIG. 7A can be weak compared with the first signals. For example, an amplitude of background noise signal 702 can be about -90 dB. The first signals in FIG. 7A include several sharp peaks 704, 706, and 708 corresponding to different optical elements in the first optical component $616_1$, and a curve 710 corresponding to an actual signal related to optical properties of sample 618. For example, peak 704 can correspond to a lens in an optical head 520 as shown in FIG. 5, due to the interference of the light reflected at a surface of the lens of the first optical component $616_1$ and the light in the reference arm. Similarly, peak 706 can correspond to mirror 542 as shown in FIG. 5, and peak 708 can correspond to other interfaces on the optical path in the first optical component $616_1$, e. g., the interface at an end of the optical fiber between air and a material of the optical fiber. Peaks 704, 706, and 708 are system-related peaks, and their appearance in the returned signal is independent of sample 618. These system-related peaks can be weak. For example, amplitude of peaks 704, 706, and 708 can be less than -55 dB. Relative positions between these system-related peaks can be stable. For example, a distance $L_{2A}$ between peaks 704 and 706, and a distance $L_{4A}$ between peaks 704 and 708, are substantially not changed when the position of the first optical component $616_1$ is changed with respect to sample 618. In some embodiments, distances $L_{2A}$ and $L_{4A}$ can be in a range of several tens of micrometers to several hundreds of micrometers. In some embodiments, data processing unit 626 can detect peaks 704, 706, and 708 using a method of peak detection.

In FIG. 7A, curve 710 is within a region $W_{2A}$, which is a region of interest including information about optical properties of the first location of sample 618 as a function of scan depth. The range of region $W_{2A}$, and its position with respect to peaks 704, 706, and 708 can be affected by complicated real-time factors, such as the optical properties of the first location on sample 618 being probed by the first optical components $616_1$ and the distance between the first optical components $616_1$ and the first location. In some embodiments, region $W_{2A}$ can have a length of several millimeters. In some embodiments, region $W_{2A}$ can have a length greater than 1 cm. In some embodiments, data processing unit 626 can crop region $W_{2A}$, using the positions of peaks 704, 706, and 708 as reference. In some embodiments, data processing unit 626 can crop region $W_{2A}$ using a method of peak detection. Because the signal effects due to the system's optical components may be known and consistent from measurement to measurement, the signal processing system can be programmed or trained to recognize the shape of the signal corresponding to the static optical components (e.g., three sequential peaks in the example of FIG. 7A), and use that shape as a reference to determine where a region of interest (e.g., peak 710) begins. This allows the system to crop the signal such that only the region of interest is further analyzed, which efficiently saves processing time by removing portions of the signal outside the region of interest, such that they are not processed. This saves computational resources by only analyzing the region of interest in the signal, rather than the entire returned signal.

FIG. 7B illustrates second signals provided by data processing unit 626 after applying fast Fourier transform on data sampled from an electrical signal provided by optical detector 622 in FIG. 6, in which the electrical signal corresponds to an optical signal returned from a second optical component $616_2$ probing a second location of sample 618. A background noise signal 712 in FIG. 7B (similar to noise signal 702 in FIG. 7A) can be weak compared with the second signals. For example, an amplitude of background noise signal 712 can about -90 dB. Similar to the first signal in FIG. 7A, the second signals in FIG. 7B include several sharp peaks 714, 716, and 718 corresponding to different optical elements in the second optical component $616_2$, and a curve 720 corresponding to an actual signal related to optical properties of sample 618. Similar to peaks 704, 706, and 708 in FIG. 7A, peaks 714, 716, and 718 are system-related peaks and independent to sample 618, and relative positions between these system-related peaks can be stable. For example, a distance $L_{2B}$ between peaks 714 and 716 and a distance $L_{4B}$ between peaks 714 and 718 can remain substantially unchanged when the position of the second optical component $616_2$ is changed with respect to sample 618. In some embodiments, the absolute position of the system-related peak 704 in FIG. 7A can be different from the absolute position of the system-related peak 714 in FIG. 7B. Similarly, the absolute position of the system-related peak 706 in FIG. 7A can be different from the absolute position of the system-related peak 716 in FIG. 7B, and the absolute position of the system-related peak 708 in FIG. 7A can be different from the absolute position of the system-related peak 718 in FIG. 7B. Because the relative positions and shapes of these peaks are substantially unchanged, even though their absolute positions may change, they can be used as references for identifying the location of a region of interest out of the entirety of the received signal.

In FIG. 7B, curve 720 is within a region $W_{2B}$, which is a region of interest including information about optical properties of the second location of sample 618 as a function of scan depth. The range of region $W_{2B}$, and its relative position with respect to peaks 714, 716, and 718 are affected by complicated real-time factors, such as the optical properties of the second location on sample 618 being probed by the second optical components $616_2$ and the distance between the second optical components $616_2$ and the second location. Comparing FIGs. 7A and 7B, the absolute position of region $W_{2A}$ in FIG. 7A can be different from the absolute position of region $W_{2B}$ in FIG. 7B. The length of region $W_{2A}$ can also be different from the length of region $W_{2B}$. In some embodiments, the length of region $W_{2B}$ can be in a range of several millimeters. In some embodiments, the length of region $W_{2B}$ can be in a range greater than 1 cm. In some embodiments, data processing unit 626 can crop region $W_{2B}$, using the positions of peaks 714, 716, and 718 as references. In some embodiments, data processing unit 626 can crop region $W_{2B}$ using the method of peak detection.

A range of scan depth of an OCT/OCR system depends on various parameters of the OCT/OCR system. In some embodiments, the range of scan depth is closely related to specifications of the coherent light source and the data acquisition unit. For example, the range of scan depth can follow a formula $d = \dfrac{\lambda^2 B}{\Delta\lambda f_s}$, in which $d$ is the range of scan depth, $\lambda$ is the central wavelength of the coherent light source (for example, coherent light source 602 in FIG. 6), $\Delta\lambda$ is the range of wavelength swept by the coherent light source, $f_s$ is the frequency of sweeping the wavelength, and $B$ is the bandwidth of the data acquisition unit (for example, data acquisition unit 624 in FIG. 6). In an illustrative example, with $\lambda \approx 1310$ nm, $\Delta\lambda \approx 42$ nm, $f_s \approx 30$ kHz, and $B \approx 100$ MHz, the range of scan depth is d $\approx 136$ mm.

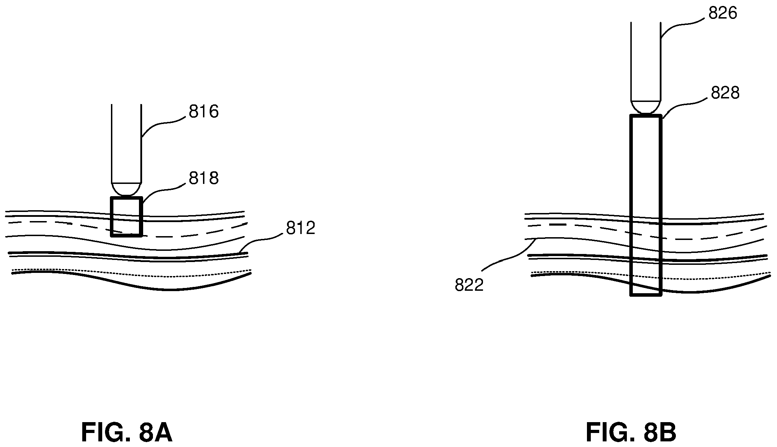

FIG. 8A and 8B illustrate diagrams of two example of OCT/OCR systems with different ranges of scan depth, according to embodiments of the present disclosure. FIG. 8A illustrates a first OCT/OCR system having a short range of scan depth, in which an optical component 816 can probe a depth range 818 into sample 812 (e. g., a lesion). In comparison, FIG. 8B illustrates a second OCT/OCR system having a long range of scan depth, in which an optical component 826 can probe a depth range 828 into sample 822 (e.g., a lesion). For example, depth range 818 in FIG. 8A can be about 1 mm or less, and depth range 828 in FIG. 8B can be 5 mm or greater. In some embodiments, a longer range of scan depth can provide more thorough information about the sample under inspection, and can also tolerate optical fibers of varying lengths. This is because the longer range of scan depth can accommodate optical fiber endpoints a range of distances from the surface of the sample, while the scan depth range still overlaps the sample region of interest. For example, optical component 826 can effectively probe sample 822 even when optical component 826 is at a distance with sample 822, since the long depth range 828 can compensate for this distance. In comparison, in order to effectively probe sample 812, optical component 816 needs to approach sample 812 at a much closer distance.

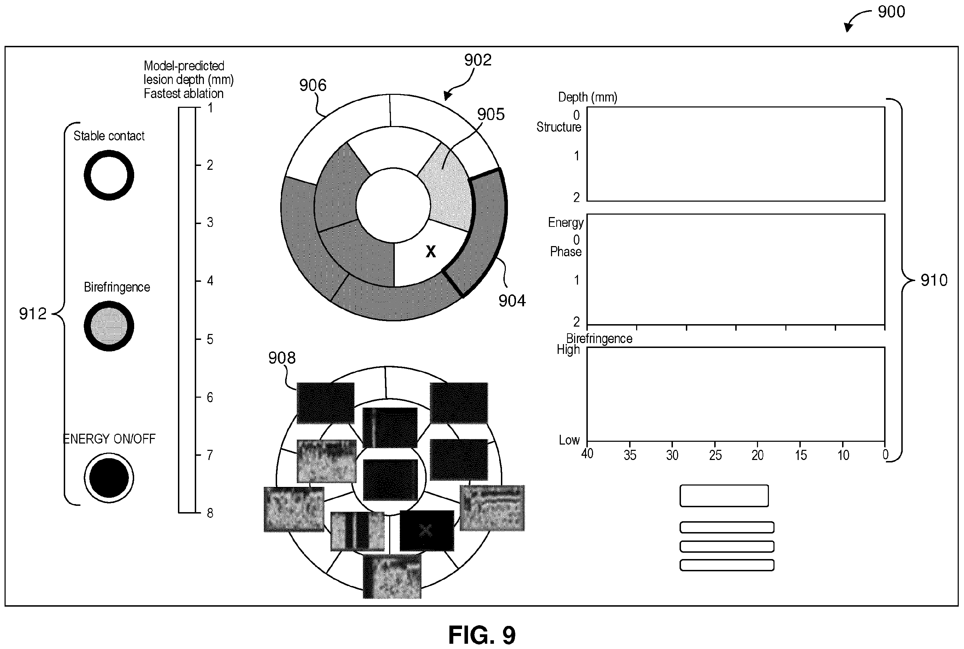

FIG. 9 illustrates an example graphical user interface (GUI) 900 showing predicted lesion depths, according to embodiments of the present disclosure. The GUI 900 provides optical measurement data, for example as processed by console 110, in real-time or near real-time for an ablation process. For example, GUI 900 may be presented on display 125 coupled to console 110 in FIG. 1. In another example, GUI 900 may be presented on the display of computer 628 in FIG. 6, according to the output of data processing unit 626. In some embodiments, the GUI 900 includes a front view 902 of the catheter tip showing different sections 904-906 corresponding to the various optical view ports in the catheter tip, each optical view ports corresponds to one of optical components (for example, optical components $616_1$-$616_N$ in FIG. 6).

In some embodiments, the front view 902 of GUI 900 may show which optical view ports of the catheter tip are in contact with tissue and which beams from the different optical view ports are in operation. For example, the dark gray sections 904 of the front view 902 may indicate a strong contact between the catheter and tissue, the light gray section 905 may indicate a minimal or intermediate contact between the catheter and tissue, and the white sections 906 may indicate no contact. In some embodiments, the different sections 904-906 may also indicate which beams are switched on or off for obtaining optical measurements from the tissue. In some embodiments, the dark gray sections 904 and light gray section 905 may indicate that the beams from the corresponding optical view ports are turned on, whereas the white sections 906 may indicate that the corresponding optical view ports are turned off.

In some embodiments, the GUI 900 may further include a plurality of tiles 908 showing the optical readout for each optical view port section in the catheter. In some embodiments, the plurality of tiles 908 may each correspond to the different sections 904-906 in the front view 902. Each tile 908 may represent the image resulting from processing, by the console, the optical signal and/or optical measurements obtained from a respective optical view port section in the catheter. In some embodiments, individual tiles 908 may be switched on or off (or may appear or disappear) based on a particular optical view port section being active at a given time. In some embodiments, the GUI 900 may include one or more graphs 910 showing ablation energy data (e.g., RF power), birefringence data, phase data, and predicted lesion depth data. In some embodiments, the GUI 900 may include one or more panels or indicators 912 that show the occurrence of a stable contact between the catheter tip and tissue, loss in birefringence, status of the ablation energy (e.g., on/off), and predicted lesion depths. In some embodiments, the GUI 900 may include one or more buttons or text boxes that allow user selection and/or customization of parameters selected for ablation or for operating the catheter during ablation.

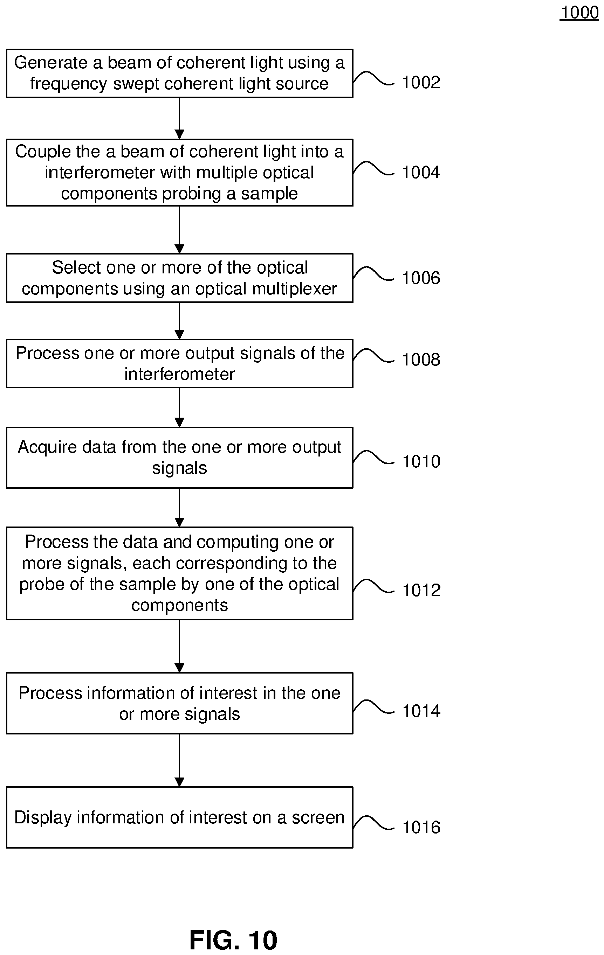

FIG. 10 illustrates an example method 1000 of conducting OCR with multiple optical components, according to embodiments of the present disclosure. In step 1002, a beam of coherent light can be generated by a coherent light source (for example, coherent light source 602 in FIG. 6). In some embodiments, the beam of coherent light can have a coherence length more than about 1 cm (single path in air) and a wavelength periodically swept within a wavelength range around a central wavelength at a wavelength sweeping frequency. In some embodiments, the coherent light source can be a tunable vertical-cavity surface-emitting laser (VCSEL), a tunable standard swept source (using, for example, a MEMS tunable filter), or an akinetic swept source. In some embodiments, the central wavelength of the beam of coherent light can be between 1000 nm and 1600 nm. For example, the central wavelength of the beam of coherent light can be about 1050 nm, 1310 nm, or 1550 nm. In some embodiments, the wavelength range swept around the central wavelength can be between 30 nm and 70 nm. For example, the wavelength range swept around the central wavelength can be about 55 nm. In some embodiments, the wavelength sweeping frequency can be between 20 kHz and 100 kHz. For example, the wavelength sweeping frequency can be about 50 kHz or 30 kHz.

In step 1004, the beam of coherent light can be coupled into an optical interferometer (for example, interferometer 610 in FIG. 6) including a reference arm and a sample arm. In some embodiments, the sample arm can include a plurality of optical paths realized by a plurality of optical components (for example, optical components $616_1$-$616_N$ in FIG. 6) inspecting a sample (for example, a lesion or a tissue under an ablation process). In some embodiments, the plurality of optical paths can have a variation of lengths within a range less than the coherence length. In some embodiment, the sample arm can include an optical multiplexer (for example, optical multiplexer 612 in FIG. 6) for selecting one or more optical paths onto which a portion of the beam of coherent light is delivered.

In step 1006, one or more optical paths can be selected using the optical multiplexer. In some embodiments, the optical multiplexer can be controlled by a control unit (for example, control unit 620 in FIG. 6) to select the one or more optical paths.

In step 1008, one or more output signals of the optical interferometer can be processed, with each of the one or more output signals corresponding to one of the plurality of optical paths. For example, the one or more output signals can be measured by an optical detector (for example, optical detector 622 in FIG. 6), which convert the one or more output signals into one or more electrical signals. In some embodiments, the one or more output signals can be amplified and/or filtered. In some embodiments, the one or more output signals can be processed within a bandwidth of about 100 MHz. For example, the optical detector can have a bandwidth of about 100 MHz.

In step 1010, data can be acquired from the plurality of output signals, for example, by sampling the one or more electrical signals at an output of the optical detector, at a data sampling rate. In some embodiments, a data acquisition unit (for example, data acquisition unit 624 in FIG. 6) can be used to acquire the data, under the data sampling rate of about 100 MHz. In some embodiments, the data sampling rate can be greater than 100 MHz. For example, the data sampling rate can be about 300 MHz. In some embodiments, the data sampling rate can be between 0 and 400 MHz. In some embodiments, the data sampling rate can be between 0 and 175 MHz.

In step 1012, the data can be processed by a data processing unit (for example, data processing unit 626 in FIG. 6). The data processing unit can compute a plurality of signals (for example, signals in FIG. 7A and 7B) from the data, and each of the plurality of signals corresponds to one of the plurality of optical paths. In some embodiments, the data processing unit can computing the plurality of signals using fast Fourier transform. In some embodiments, the plurality of signals can be optical properties of the sample as functions of depths inside the sample. In some embodiments, the plurality of signals can have depth ranges greater than about 1 cm.

In step 1014, information of interest in the plurality of signals can be processed by the data processing unit. In some embodiment, the data processing unit can detect one or more reference features (for example, peaks 704, 706, and 708 in FIG. 7A and peaks 714, 716, and 718 in FIG. 7B) in the plurality of signals, and can crop regions of interest in the plurality of signals, based on the one or more reference features. In some embodiments, the data processing unit can implement a method of peak detection to detect the one or more reference features and the regions of interest.

In step 1016, the regions of interest can be displayed as a plurality of tomographic images on a screen (for example, screen 125 in FIG. 1 or the screen of computer 628 in FIG. 6) for analysis and/or diagnosis of the sample. Regions of interest corresponding to different optical components can be displayed on different sections (for example, sections 904-906 in FIG. 9) on the screen in a real time basis.

It is to be appreciated that the Detailed Description section, and not the Summary and Abstract sections, is intended to be used to interpret the claims. The Summary and Abstract sections may set forth one or more but not all exemplary embodiments of the present disclosure as contemplated by the inventor(s), and thus, are not intended to limit the present disclosure and the appended claims in any way.

Embodiments of the present disclosure have been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific embodiments will so fully reveal the general nature of the disclosure that others can, by applying knowledge within the skill of the art, readily modify and/or adapt for various applications such specific embodiments, without undue experimentation, without departing from the general concept of the present disclosure. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

The breadth and scope of the present disclosure should not be limited by any of the above-described exemplary embodiments, but should be defined only in accordance with the following claims and their equivalents.

Moreover, the following aspects shall be considered as a part of the overall disclosure in the context of the present invention:

1. A method of performing optical coherence tomography, comprising:

generating a beam of coherent light having a coherence length more than about 1 cm and a wavelength periodically swept within a wavelength range around a central wavelength at a wavelength sweeping frequency;
coupling the beam of coherent light into an optical interferometer comprising a reference arm and a sample arm, wherein the sample arm comprises:

an optical multiplexer; and
a plurality of optical paths with a variation of lengths within a range less than the coherence length; selecting an optical path of the plurality of optical paths using the optical multiplexer;
processing a plurality of output signals of the optical interferometer, wherein each of the plurality of output signals corresponds to one of the plurality of optical paths;
acquiring data from the plurality of output signals at a data sampling rate; and
processing the data and generating a plurality of signals from the data, wherein the plurality of signals have depth ranges of at least about 1 cm, and wherein each of the plurality of signals corresponds to one of the plurality of optical paths.

2. The method of aspect 1, further comprising:

detecting one or more reference features in the plurality of signals; and

cropping regions of interest in the plurality of signals, based on the one or more reference features.

3. The method of aspect 1 or 2, wherein the beam of coherent light is generated by a tunable vertical-cavity surface-emitting laser (VCSEL) or an akinetic swept source.

4. The method of any of the preceding aspects, wherein:

the central wavelength of the beam of coherent light is between 1000 nm and 1600 nm; and the wavelength range swept around the central wavelength is between 30 nm and 70 nm.

5. The method of any of the preceding aspects, wherein:

the central wavelength of the beam of coherent light is about 1310 nm; and the wavelength range swept around the central wavelength is about 55 nm.

6. The method of any of the preceding aspects, wherein the wavelength sweeping frequency is between 20 and 100 kHz.

7. The method of any of the preceding aspects, wherein the wavelength sweeping frequency is about 50 kHz.

8. The method of any of the preceding aspects, wherein the sample arm further comprises a plurality of optical fibers, and wherein each of the plurality of optical paths comprises one of the plurality of optical fibers.

9. The method of any of the preceding aspects, wherein processing the plurality of output signals of the optical interferometer comprises measuring and amplifying the plurality of output signals within a bandwidth of about 100 MHz.

10. The method of any of the preceding aspects, wherein the data sampling rate is between 0 and 400 MHz.

11. The method of any of the preceding aspects, wherein the data sampling rate is between 0 and 175 MHz.

12. The method of any of the preceding aspects, wherein computing the plurality of signals from the data comprises processing the data using fast Fourier transform.

13. The method of any of the preceding aspects, further comprising displaying a plurality of tomographic images on a screen.

14. The method of any of the preceding aspects, wherein the coherence length of the beam of coherent light is about 10 cm.

15. A system of optical coherence tomography, comprising:

a light source configured to output a beam of coherent light having a coherence length more than about 1 cm and a wavelength periodically swept within a wavelength range around a central wavelength at a wavelength sweeping frequency;
an optical interferometer, comprising:

an input coupled to the beam of coherent light;
an output;
a reference arm; and
a sample arm, comprising a plurality of optical paths with a variation of lengths within a range less than the coherence length;
an optical multiplexer configured to select one of the plurality of optical paths;
an optical detector coupled to the output;
a data acquisition unit configured to acquire data from the optical detector at a data sampling rate; and
a data processing unit configured to generate a plurality of signals from the data, wherein the plurality of signals have depth ranges of at least 1 cm, and wherein each of the plurality of signals corresponds to one of the plurality of optical paths.

16. The system of aspect 15, wherein the data processing unit is further configured to:

detect one or more reference features in the plurality of signals; and
crop regions of interest in the plurality of signals, based on the one or more reference features.

17. The system of aspects 15 or 16, wherein the light source is a tunable VCSEL or an akinetic swept source.

18. The system of any of aspects 15-17, wherein:

the central wavelength of the beam of coherent light is between 1000 nm and 1600 nm; and
the wavelength range swept around the central wavelength is between 30 nm and 70 nm.

19. The system of any of aspects 15-18, wherein:

the central wavelength of the beam of coherent light is about 1310 nm; and
the wavelength range swept around the central wavelength is about 55 nm.

20. The system of any of aspects 15-19, wherein the wavelength sweeping frequency is between 20 and 100 kHz.

21. The system of any of aspects 15-20, wherein the wavelength sweeping frequency is about 50 kHz.

22. The system of any of aspects 15-21, wherein each of the plurality of optical paths comprises an optical fiber.

23. The system of any of aspects 15-22, wherein the data sampling rate is between 0 and about 400 MHz.

24. The system of any of aspects 15-23, wherein the data sampling rate is between 0 and about 175 MHz.

25. The system of any of aspects 15-24, wherein the optical detector is configured to measure and amplify an output signal of the output within a bandwidth of about 100 MHz.

26. The system of any of aspects 15-25, wherein the data processing unit generates the plurality of signals from the data using fast Fourier transform.

27. The system of any of aspects 15-26, further comprising a screen configured to display a plurality of tomographic images, based on the generated plurality of signals.

**Claims**

1. A system of optical coherence tomography, comprising:

a light source configured to output a beam of coherent light having a coherence length more than about 1 cm and a wavelength periodically swept within a wavelength range around a central wavelength at a wavelength sweeping frequency;
an optical interferometer, comprising:

an input coupled to the beam of coherent light;
an output;
a reference arm; and
a sample arm, comprising a plurality of optical paths with a variation of lengths within a range less than the coherence length;

an optical multiplexer configured to select one of the plurality of optical paths;
an optical detector coupled to the output;
a data acquisition unit configured to acquire data from the optical detector at a data sampling rate; and
a data processing unit configured to generate a plurality of signals from the data, wherein the plurality of signals have depth ranges of at least 1 cm, and wherein each of the plurality of signals corresponds to one of the plurality of optical paths.

2. The system of claim 1, wherein the data processing unit is further configured to:

detect one or more reference features in the plurality of signals; and
crop regions of interest in the plurality of signals, based on the one or more reference features.

3. The system of the preceding claims, wherein the light source is a tunable VCSEL or an akinetic swept source.

4. The system of any of the preceding claims, wherein:

the central wavelength of the beam of coherent light is between 1000 nm and 1600 nm; and
the wavelength range swept around the central wavelength is between 30 nm and 70 nm.

5. The system of any of the preceding claims, wherein:
the central wavelength of the beam of coherent light is about 1310 nm; and
the wavelength range swept around the central wavelength is about 55 nm.

6. The system of any of the preceding claims, wherein the wavelength sweeping frequency is between 20 and 100 kHz.

7. The system of any of the preceding claims, wherein the wavelength sweeping frequency is about 50 kHz.

8. The system of any of the preceding claims, wherein each of the plurality of optical paths comprises an optical fiber.

9. The system of any of the preceding claims, wherein the data sampling rate is between 0 and about 400 MHz.

10. The system of any of the preceding claims, wherein the data sampling rate is between 0 and about 175 MHz.

**11.** The system of any of the preceding claims, wherein the optical detector is configured to measure and amplify an output signal of the output within a bandwidth of about 100 MHz.

**12.** The system of any of the preceding claims, wherein the data processing unit generates the plurality of signals from the data using fast Fourier transform.

**13.** The system of any of the preceding claims, further comprising a screen configured to display a plurality of tomographic images, based on the generated plurality of signals.

FIG. 1

204 — Distal Part

206 — Sheath

202 — Proximal Part

208 — Processing Device

210

200

**FIG. 2**

**FIG. 3A**

**FIG. 3B**

FIG. 4

**FIG. 5**

EP 4 390 300 A1

FIG. 6

FIG. 7A

FIG. 7B

FIG. 8B

FIG. 8A

FIG. 9

1000

```
Generate a beam of coherent light using a
frequency swept coherent light source          1002

                    ↓

Couple the a beam of coherent light into a
interferometer with multiple optical            1004
components probing a sample

                    ↓

Select one or more of the optical
components using an optical multiplexer         1006

                    ↓

Process one or more output signals of the       1008
interferometer

                    ↓

Acquire data from the one or more output
signals                                         1010

                    ↓

Process the data and computing one or
more signals, each corresponding to the
probe of the sample by one of the optical       1012
components

                    ↓

Process information of interest in the one
or more signals                                 1014

                    ↓

Display information of interest on a screen     1016
```

FIG. 10

Europäisches
Patentamt

European
Patent Office

Office européen
des brevets

# EUROPEAN SEARCH REPORT

Application Number

EP 22 38 3257

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/231877 A1 (CARESTREAM DENTAL LLC [US]) 3 November 2022 (2022-11-03) * paragraphs [0001], [0044], [0046], [0051], [0066] – [0077]; figures 1,5,6 * | 1,2,5,7, 9-13 | INV. G01B9/02015 G01B9/02091 |
| X | CHAO ZHOU ET AL: "Space-division multiplexing optical coherence tomography", OPTICS EXPRESS, vol. 21, no. 16, 12 August 2013 (2013-08-12), page 19219, XP055147802, ISSN: 1094-4087, DOI: 10.1364/OE.21.019219 * chapter 1; figure 1 * | 1,3,5-11 | |
| A | US 2014/078510 A1 (RUBIO GUIVERNAU JOSE LUIS [ES] ET AL) 20 March 2014 (2014-03-20) * paragraphs [0039], [0048] * | 1 | |
| A | US 2021/212569 A1 (SANCHO DURÁ JUAN [ES] ET AL) 15 July 2021 (2021-07-15) * figures 1-5 * | 1-13 | **TECHNICAL FIELDS SEARCHED (IPC)** G01B |

The present search report has been drawn up for all claims

| Place of search | Date of completion of the search | Examiner |
|---|---|---|
| Munich | 17 May 2023 | Biedermann, Benjamin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...............................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 38 3257

This annex lists the patent family members relating to the patent documents cited in the above-mentioned European search report.
The members are as contained in the European Patent Office EDP file on
The European Patent Office is in no way liable for these particulars which are merely given for the purpose of information.

17-05-2023

| Patent document cited in search report | | Publication date | Patent family member(s) | | Publication date |
|---|---|---|---|---|---|
| WO 2022231877 | A1 | 03-11-2022 | NONE | | |
| US 2014078510 | A1 | 20-03-2014 | AU | 2012260954 B2 | 18-06-2015 |
| | | | BR | 112013029785 A2 | 17-01-2017 |
| | | | CA | 2836609 A1 | 29-11-2012 |
| | | | CN | 103688133 A | 26-03-2014 |
| | | | EP | 2710327 A1 | 26-03-2014 |
| | | | ES | 2415555 A1 | 25-07-2013 |
| | | | JP | 6118796 B2 | 19-04-2017 |
| | | | JP | 2014517286 A | 17-07-2014 |
| | | | US | 2014078510 A1 | 20-03-2014 |
| | | | WO | 2012160005 A1 | 29-11-2012 |
| US 2021212569 | A1 | 15-07-2021 | CA | 3162823 A1 | 22-07-2021 |
| | | | CN | 115003213 A | 02-09-2022 |
| | | | DE | 21702166 T1 | 23-03-2023 |
| | | | EP | 4090232 A1 | 23-11-2022 |
| | | | JP | 2023510828 A | 15-03-2023 |
| | | | US | 2021212569 A1 | 15-07-2021 |
| | | | WO | 2021144318 A1 | 22-07-2021 |

EPO FORM P0459

For more details about this annex : see Official Journal of the European Patent Office, No. 12/82